# EUROPEAN PATENT SPECIFICATION

(11) **EP 1 541 102 B1**
(45) Date of publication and mention of the grant of the patent: **05.10.2011**
(21) Application number: 03104594.1
(22) Date of filing: 08.12.2003
(51) Int. Cl.: A61F 13/15, A61F 15/00

(54) **Package kit for personal care articles**
Verpackungszusammenstellung für Hygieneartikel
Kit d'emballage pour article de soins hygiènique

(43) Date of publication of application: 15.06.2005
(73) Proprietor: THE PROCTER & GAMBLE COMPANY, Cincinnati, OH 45202 (US)
(72) Inventor: Hodges, Jonathan Norman Robert, Windsor, Berkshire SL4 5NF (GB); Kohlweyer, Christian, 61118 Bad Vilbel (DE)
(74) Representative: Briatore, Andrea

(56) References cited:
- EP-A- 0 930 052
- WO-A-00/35776
- WO-A-02/094679
- WO-A-02/096331
- WO-A-03/050011
- US-A- 5 242 057
- US-A- 5 259 503
- US-A- 6 073 833
- US-A1- 2002 148 749
- US-A1- 2003 102 238
- US-A1- 2003 130 632
- US-A1- 2004 064 122

## Description

### FIELD OF THE INVENTION

The present invention relates to a package kit for packaging personal care articles, especially sanitary absorbent articles of personal hygiene. The package kit comprises a display stand and a container containing at least one personal care article. The container is removably insertable into the display stand, which supports the container to stand upright.

### BACKGROUND OF THE INVENTION

Personal care articles, such as absorbent articles like sanitary napkins or panty liners are known in the art since long. As these articles have to fulfil a hygienic purpose in close contact to the human body it is clear that such articles have to be packaged in a hygienically satisfactory manner. In particular, the packaging of such articles has to prevent contamination of the packaged articles from dust, moisture and preferably also microorganisms, at least during the shelf life of these articles.

There are many references in the art for addressing this problem. An example is EP -A-930,052, which discloses a package for a stack of absorbent articles. The articles are stacked on top of each other by fastening the adhesively-coated backsheet onto the topsheet of an underlying article. The so-obtained stack is then inserted into a carton, which has a release coating on its inside surface. By this way the release paper covering the adhesive on the backsheet of the articles becomes obsolete. However, such a stack in a carton is highly accessible to dust, moisture or humidity and the articles of the stack are thus only very poorly protected.

Another reference disclosing a package for absorbent articles is WO-A-02/36443. This document discloses a stack of sanitary napkins or panty liners, which is inserted into a flexible package member, such as a pouch or a carton, for better hygienic protection when carried in the bag of a woman. In particular, such a package is disclosed to protect the articles from being contaminated by sharp or edgy things like pens etc., which are also in the back. However, the package of WO -A-02/36443 is not able to stand in a self-supported manner, especially when articles have been taken out of the package member, due to the flexible material it is made of. This is particularly disadvantageous at the user's home, since in typical bathrooms, where articles like sanitary napkins or panty liners are conventionally stored, storage space is limited. Thus, a package member, which is not able to stand self-supported because it is not able to maintain a certain shape will require unnecessarily much space and will thus be not satisfactory to consumers. The same consideration is applicable to the shelves in a retail store, where packages, which can stand upright in a self-supported manner require less space than packages, which are not capable to do so.

The document WO-A-02/39943 discloses a package for absorbent articles. The package, which is a flexible pouch, is intended to contain a plurality of absorbent artic les and is also provided with a re-closable opening. However, due to the construction of the package of WO-A-02/39943 out of flexible material, the same disadvantages as already discussed in the preceding paragraph are associated with it, too.

All these exemplary prior art references have certain drawbacks. In particular, there is no package for personal care articles, which is on one hand suitable for use at home as a convenient and space efficient supply for such articles and which is on the other hand also suitable for being carried in a woman's bag in a convenient and hygienically satisfying manner.

Thus, there exists the need for a package for personal care articles, particularly sanitary absorbent articles such as sanitary napkins or panty liners, which protects the packaged articles from contamination, which is efficient in storage space consumption both at home and at the Point-of Sale, which provides the user with increased flexibility of use and which can be produced at low cost.

### SUMMARY OF THE INVENTION

The present invention provides a package kit comprising a display stand and a container. The container is removably insertable into the display stand and is sized and shaped for containing multiple personal care articles, such as absorbent articles like sanitary napkins or panty liners. The container is typically not capable to stand upright on its own, i.e. to maintain an upright position in a self-supported manner. The display stand is suited for receiving at least one such container. The display stand supports the container such that the container is enabled to maintain an upright position.

It has been found that the package kit of the present invention offers better protection of the articles contained therein as well as better stability on the shelf or in the user's cupboard compared to conventional pouch packages. Additionally, the package kit of the present invention requires less space on the shelf or in the user's cupboard compared to conventional pouch packages, as it stands upright, preferably with its largest dimension being oriented vertically. Furthermore, it provides better hygienic protection to the articles contained therein compared to conventional carton packages, since the container according to the present invention is sealing the packaged articles from the outside environment much more efficiently than a carton, especially when the carton is opened. Moreover, the package kit of the present invention can be produced at lower cost compared to conventional carton packages.

A further advantageous feature of the package kit of the present invention is that it is refillable. This means that it is possible to discard an empty container and insert a new one into the display stand.

A further advantage of the package kit of the present invention is its flexibility of use. The combination of the display stand and the container ensures that the container, even when more and more articles are being taken out of it and thus the flexibility of the whole container increases, is still standing upright and thus requires not more space in the user's bathroom cupboard than necessary. On the other side, the user can take the container out of the display stand and can conveniently transport the container in her hand bag for out-of-home use. Consequently, the user has complete flexibility of use of the personal care articles contained in the package kit of the present invention.

### BRIEF-DESCRIPTION OF THE DRAWINGS

Figure 1 illustrates an embodiment of the package kit of the present invention, wherein the display stand is substantially cylindrical with an ellipsoidal cross-section and is furthermore open on both ends.
Figure 2 illustrates an embodiment of the package kit of the present invention, wherein the display stand has a frustopyramidal shape and a rectangular cross -section and is open on both ends.
Figure 3 illustrates an embodiment of the package kit of the present invention, wherein the display stand comprises two plates, which are fixed to a bottom and betwe en which the container is held.
Figure 4 illustrates an embodiment of the package kit of the present invention, wherein the display stand has a cylindrical shape and a double-edged, pseudo-ellipsoidal cross-section and is closed at its lower end.

### DETAILED DESCRIPTION OF THE INVENTION

'Rigid' as used herein refers to materials with at least some flexural stiffness, i.e. a significant degree to maintain their original shape. Exemplary rigid materials in the context of the present invention are carton paper, carton board, card board, enforced paper, corrugate or the like or combinations thereof. Further examples of rigid materials for use herein are plastic or rubber -like polymeric materials with a significant degree to maintain their shape.

In contrast to the preceding paragraph, materials, which have no significant degree to maintain their shape because they have no substantial inherent flexural stiffness, are called 'flexible' herein. Typical flexible materials suitable for use herein are thin polymeric films or nonwoven materials or combinations thereof.

'Personal care articles' as used herein means substantially flat cosmetic articles like cosmetic wipes or pads, or substantially flat hygienic absorbent articles, such as sanitary napkins, panty liners or incontinence inserts, tissues, handkerchiefs or toilet paper. The present invention is particularly suitable for disposable absorbent articles for feminine hygiene, preferably sanitary napkins, panty liners, tampons or incontinence pads. Other suitable absorbent articles are perspiration pads like underarm sweat pads or hat bands.

The term 'absorbent article' is used herein in a very broad sense including any article being able to receive and/or absorb and/or contain and/or retain fluids and/or exudates, especially bodily fluids/bodily exudates. Absorbent articles for feminine hygiene, as referred to in the present invention typically comprises a fluid pervious topsheet as the wearer-facing layer, a fluid impervious backsheet as the garment-facing layer that is preferably water vapour and/or gas pervious and an absorbent core comprised there between. Furthermore, absorbent articles for feminine hygiene in the context of the present invention are provided with a means for their attachment to the user's garment, in particular with an adhesive. Particularly preferred absorbent articles in the context of the present invention are disposable absorbent articles.

The term 'disposable' is used herein to describe articles, which are not intended to be laundered or otherwise restored or reused as an article (i.e. they are intended to be discarded after a single use and preferably to be recycled, composted or otherwise disposed of in an environmentally compatible manner).

'Upright position' herein means a position in which the container according to the present invention is in a position in which the smallest of its length-, width- and thickness dimensions is extending substantially parallel to a substantially even horizontal surface. As an illustrative examp le, a typical pouch as a non-limiting example of the container according to the present invention has two coextensive major surfaces, which are separated from each other by the thickness dimension of the pouch. Both surfaces are connected along their peripheral edges. The longer ones of these peripheral edges define the length dimension of the pouch and the shorter ones the width dimension. An upright position of the pouch on a substantially even horizontal surface according to the definition herein would be given in case the pouch is in a position, in which its length - or its width dimension lies in the plane of said horizontal surface; in other words, if the pouch stands on one of its peripheral edges.

Maintaining a position in a 'self-supported' manner herein means that the container according to the present invention can maintain this position, such as an upright standing position, without any external support.

The package kit (1) of the present invention is constituted by the combination of a display s tand (2) and a flexible container (3), which is removably insertable into said display stand (2).

The display stand (2) of the package kit (1) of the present invention is suited for insertion of the container (3) and for supporting the container (3) to stand in an upright position, preferably with the container's greatest dimension being oriented horizontally. The display stand (2) can have various configurations.

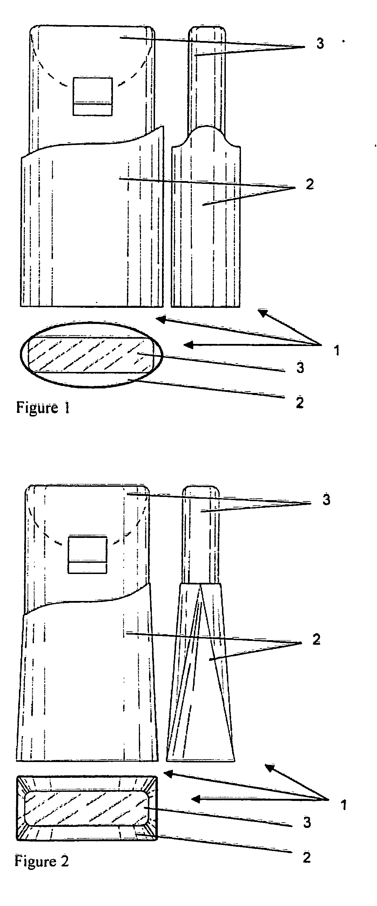

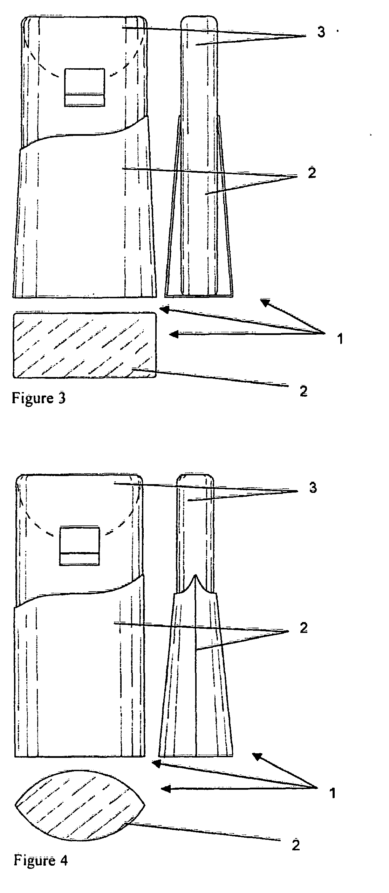

In one embodiment herein the display stand (2) herein is substantially cylindrical with an essentially circular or ellipsoidal cross-section. This is illustrated in Figure 1. One of the openings at the ends of the cylinder can optionally be closed with a flat bottom, on which the display stand then stands, as illustrated in Figure 4. The remaining opening serves then as the insertion opening for insertion of the container (3). In other embodiments herein the display stand (2) has a substantially square or rectangular cross-section. Generally, there is basically no limitation in cross -sectional shape of the display stand (2), as long as the display stand (2) is sized and shaped to allow removable insertion of the container (3).

In another embodiment, which is illustrated in Figure 3, the display stand (2) is constituted by two plates, which are spaced apart from each other such that the container can be fixated between them. Preferably, the plates are connected to each other at their lower ends.

Generally, the display stand can be made of any material, which is rigid enough to enable the display stand to support a container containing at least one personal care article to maintain an upright position, when said container is inserted into said display stand. Materials, which are preferred in the context of the present invention, are carton board, car d board, enforced paper, corrugate or the like. Furthermore, the display stand can preferably also be made of plastic or rubber-like polymeric material.

It is preferred in the present invention that the shape of the opening of the display stand (2) essentially equals the shape of the bottom. It is further preferred that the opening and the bottom of the display stand (2) are substantially co-extensive, spaced apart by the side walls of the display stand. Such embodiments are illustrated in Figures 1 and 4. However, it is also within the scope of the present invention that the opening and the bottom of the display stand (2) are not co -extensive, such as in case the side walls of the display stand (2) have different heights, or in case one or more side walls have a variable height, or in case the sidewalls have an angle other than 90° with respect to a substantially even horizontal surface the display stand is placed on or have different angles for each side wall. Figure 2 illustrates embodiments with a display stands of frustopyramidal shape.

It is furthermore within the scope of the present invention that the cross-section of the display stand is variable with its height. Thus, shallow containers may be fixed safely by a narrow cross section at the insertion opening of the display stand and at the same time stability of the overall package can be maximised by a larger cross section at the bottom of the display stand. In an embodiment herein the bottom of the display stand has a square shape, whereas the insertion opening has a substantially ellipsoidal shape. Also the reverse configuration, i.e. circular or ellipsoidal bottom and square insertion opening are within the scope of the present invention. It will be apparent to the skilled artisan that also numerous other different configurations of bottom and opening are possible, which will lead to a variable cross-sectional shape of the display stand. Such an embodiment is illustrated in Figure 2.

In other embodiments the display stand herein is provided with windows, through which the container can be seen from outside. These windows can be provided by holes in the sidewalls of the display stand or by zones in the sidewalls, which are transparent.

According to further embodiments herein the display stand accord ing to the present invention is provided with means for aiding collapsibility of said display stand. This is beneficial for improving the disposability of the display stand, since it requires less space in the garbage container in collapsed condition. Means for providing collapsibility are for instance cuts or weakness lines, such as perforation lines or score lines.

The sidewalls of the display stand herein have a height of at least 10%, preferably at least 25% and more preferably at least 50% of the vertical extension of the container once the container is inserted into the display stand. However, it is also preferred that the height of the sidewalls of the display stand are as high as or even exceed the vertical extension of the container once the container is inserted into the display stand, because this would render the container less accessible to the user.

The flexible container of the package kit of the present invention has an interior sized and shaped for receiving multiple personal care articles, therein. The personal care articles can be either folded or unfolded. The container is fully enclosing the personal care articles.

The container herein can be made of any suitable flexible material, which pr otects the personal care articles from being contaminated by dust or moisture. Suitable materials are e.g. plastic films like films made of polyvinyl chloride, polyethylene, polypropylene, polyethylene terephtalate (PET) and other polymers, film laminates of any combination of the said polymers or the like. Other suitable materials for making the container are e.g. metallised plastic films, laminates of films and nonwovens or paper with coatings for imparting moisture-barrier properties to it.

The container herein can have a variety of shapes. It can be a classical "pouch" container. A pouch container has two substantially coextensive surfaces, which are connected along their peripheral edges and spaced apart by a hollow interior. In other embodiments herein, the container has a more parallelepipedal shape, where two substantially parallel and coextensive main surfaces are connected along their edges by four minor surfaces, which are all oriented essentially perpendicular to said main surfaces. The space enclosed by all these surfaces is the hollow interior of this container.

Due to the nature of the container, which is made of flexible material, and the nature of the personal care articles contained therein, which are typically also substantially soft and flexible, the container will not or only to an insufficient degree be able to maintain an upright position as defined herein. Because of its flexibility and softness, which even more increases when more and more articles have been taken out of the container, it will rather fall over when it is unsupported and assume a position, where the largest surface lies in the plane of the surface it is located on.

The container herein must be opened by the user for accessing the articles contained therein. Therefore, the container according to the present invention is preferably provided with a means to assists opening of said container. Such a means can be comprised of one or more weakness lines or perforation lines. An example for an opening aid comprised of a perforation line can be found at the package of Tempo^{®} handkerchiefs. Other possibilities of openings means for use herein are overarching flaps, such as envelopes sealed in a semi-permanent way, by e.g. adhesives tapes.

In another preferred embodiment the container herein is provided with a re-closable opening. This can be achieved by providing the opening with e.g. an adhesive tape, which is permanently attached to the first side of the opening and removably attached to the second side of the opening. Thus, when removing the adhesive tape from the second side of the opening said opening becomes accessible. Thereafter, by re-attaching the adhesive tape to the second side of the opening said opening becomes closed again.

A particularly preferred configuration of the container herein is the classical "pouch" configuration. Such a pouch-like container can be obtained by starting from a substantially cylindrical, pipe -like segment of material, e.g. plastic film, into which a one or more personal care articles are in serted. After this step both open sides of the cylindrical film segment are closed. Thereby the articles are completely enclosed by the film. Such pipe-like film segments can be made by a wrap-around or a flow-wrap process or by folding a square or rectangular piece of film onto itself, such that two parallel edges of the piece of film overlap. The film is then sealed to itself along this area of overlap. The so-obtained container is substantially rectangular and has a length, a width and a thickness. Another possibility to form a pouch container is to fold a substantially rectangular piece of material onto itself, such that the line of folding is dividing this piece of material into two segments with substantially the same size and shape in a way that both segments are essentially coextensive after this folding operation. By sealing the three open edges of the folded piece of material a pouch container is formed. Another possibility of foaming a pouch container is by placing two identically sized and shaped pieces of material onto each other in a coextensive manner and sealing them to each other along their peripheral edge. The sealing operations referred to in this paragraph are preferably carried out by adhesives, by heat or ultrasonic bonding, or by crimpi ng.

In a further embodiment of the present invention the display stand is suited for insertion of more than one container.

In a particular preferred embodiment of the package kit of the present invention the container is of the pouch type and made of polyethylene film. The container contains a stack of 20 panty liners, which are unfolded and which are stacked on top of each other in a substantially coextensive manner. The container is sealed around the stack of panty liners, such that the stack is completely enclosed. The shape of the container is primarily influenced by the shape of the stack of absorbent thickness, which essentially have the same dimensions as the length, height and width of the stack of absorbent articles. The container of this embodiment is provided with a perforation line on one of its ends for ease of opening. The so-formed opening can be re-closed by a piece of adhesive tape, which is permanently attached to the outside surface of the container on one side of the perforation line and which is removably attached to the outside surface of the container on the other side of the perforation line. The display stand of this embodiment is made of carton board material and has an ellipsoidal bottom and a sidewall with an essentially ellipsoidal cross-section. Also the insertion opening is substantially ellipsoidal. The height of the side wall is about 50% of the length of the container. Due to this the container extends beyond the sidewall and can thus be easily grasped by the user.

In a further preferred embodiment of the present invention the container is not provided with any information visible from outside, which allows identification of the personal care articles it contains. Product-specific information visible to outside is only to be found on the display stand. When a user is going out and only carries the container with her in her hand bag she is provided with high discretion concerning the kind of articles contained in the container. This is especially critical for absorbent articles of feminine hygiene, which are usually not intended to be exposed to the public by the user.

The citation of any document is not to be construed as an admission that it is prior art with respect to the present invention.

## Claims

1. Package kit (1) for personal care articles, **characterized in that** said package kit comprises a display stand (2) and at least one flexible container (3), said container (3) being comprised of substantially flexible material selected from film or nonwoven materials or combinations thereof, said container being removably insertable into said display stand (2), said container (3) having a length dimension, a width dimension and a thickness dimension, said container (3) further having a hollow interior comprising multiple personal care articles, said container (3) having at least one upright position wherein the smallest of said dimensions of said container (3) is oriented parallel with respect to a even horizontal surface, wherein said container (3) is not capable of maintaining said upright position in a self-supported manner, said display stand (2) being suited for receiving said container(3), said display stand (2) supporting said container (3) to maintain said upright position when said container is inserted in to said display stand (2) wherein said display stand (2) is supporting said container(3) to maintain a position in which the largest of said dimensions of said container (3) is oriented vertically with respect to a even horizontal surface.

2. The package kit of any of the previous claims, **characterized in that** said display stand comprises at least one side wall.

3. The package kit of claim 3, **characterized in that** said sidewall has a height, which is lower than the greatest dimension of said container.

4. The package kit (1) of any of the previous claims, **characterized in that** said display stand (2) is comprised of relatively rigid material, which is preferably being selected from carton paper, carton board, card board, enforced paper, corrugate or the like, or plastic or rubber-like polymeric material, or combinations thereof.

5. The package kit (1) of any of the previous claims, **characterized in that** said display stand (2) comprises zones through which the container (3) is visible.

6. The package kit (1) of any of the previous claims, **characterized in that** said display stand (2) is provided with means aiding disposability by making said display stand (2) collapsible, said means preferably being selected from weakness lines, score lines or semi-permanent adhesive joints.

7. The package kit (1) of any of the previous claims, **characterized in that** said container (3) is free of any information allowing identification of the products it contains and product information is only to be found on said display stand (2).

8. The package kit (1) of any of the previous claims, **characterized in that** said container (3) comprises means for facilitating opening said container.

9. The package kit (1) of any of the previous claims, **characterized in that** said container (3) is re-closable.

10. The package kit (1) of any of the previous claims, wherein said package kit consists of one display stand (2) and one container (3).

11. The package kit (1) of any of the preceding claims, wherein said personal care articles are disposable absorbent articles for feminine hygiene, preferably tampons, pantiliner, sanitary napkin or incontinent pads.

## Patentansprüche

1. Verpackungsset (1) für Körperpflegeartikel, **dadurch gekennzeichnet, dass** das Verpackungsset einen Schauständer (2) und mindestens einen flexiblen Behälter (3) umfasst, wobei der Behälter (3) aus im Wesentlichen flexiblem Material besteht, das ausgewählt ist aus Folie oder Vliesmaterialien oder Kombinationen davon, wobei der Behälter in den Schauständer (2) entfernbar einsetzbar ist, wobei der Behälter (3) eine Längenabmessung, eine Breitenabmessung und eine Dickenabmessung aufweist, wobei der Behälter (3) ferner einen hohlen Innenraum aufweist, der eine Vielzahl von Körperpflegeartikeln umfasst, wobei der Behälter (3) mindestens eine aufrechte Position aufweist, wobei die kleinste der Abmessungen des Behälters (3) parallel zu einer ebenen horizontalen Oberfläche ausgerichtet ist, wobei der Behälter (3) die aufrechte Position nicht selbsttragend beibehalten kann, wobei der Schauständer (2) geeignet ist, den Behälter (3) aufzunehmen, wobei der Schauständer (2) den Behälter (3) stützt, um die aufrechte Position beizubehalten, wenn der Behälter in den Schauständer (2) eingesetzt ist, wobei der Schauständer (2) den Behälter (3) stützt, um eine Position beizubehalten, in der die größte der Abmessungen des Behälters (3) zu einer ebenen horizontalen Oberfläche vertikal ausgerichtet ist.

2. Verpackungsset nach einem der vorstehenden Ansprüche, **dadurch gekennzeichnet, dass** der Schauständer mindestens eine Seitenwand umfasst.

3. Verpackungsset nach Anspruch 3, **dadurch gekennzeichnet, dass** die Seitenwand eine Höhe aufweist, die niedriger als die größte Abmessung des Behälters ist.

4. Verpackungsset (1) nach einem der vorstehenden Ansprüche, **dadurch gekennzeichnet, dass** der Schauständer (2) aus relativ steifem Material besteht, das vorzugsweise ausgewählt ist aus Kartonpapier, Karton, Pappe, verstärktem Papier, Wellpappe oder dergleichen oder kunststoff- oder kautschukähnlichem Polymermaterial oder Kombinationen davon.

5. Verpackungsset (1) nach einem der vorstehenden Ansprüche, **dadurch gekennzeichnet, dass** der Schauständer (2) Zonen umfasst, durch welche der Behälter (3) sichtbar ist.

6. Verpackungsset (1) nach einem der vorstehenden Ansprüche, **dadurch gekennzeichnet, dass** der Schauständer (2) mit Anordnungshilfsmitteln versehen ist, die den Schauständer (2) zusammenklappbar machen, wobei die Mittel vorzugsweise ausgewählt sind aus Schwächungslinien, Rillenlinien oder semipermanenten Klebeverbindungen.

7. Verpackungsset (1) nach einem der vorstehenden Ansprüche, **dadurch gekennzeichnet, dass** der Behälter (3) frei von Informationen ist, die eine Identifizierung der darin enthaltenen Produkte ermöglichen, und Produktinformationen nur auf dem Schauständer (2) zu finden sind.

8. Verpackungsset (1) nach einem der vorstehenden Ansprüche, **dadurch gekennzeichnet, dass** der Behälter (3) Mittel zum Ermöglichen des Öffnens des Behälters umfasst.

9. Verpackungsset (1) nach einem der vorstehenden Ansprüche, **dadurch gekennzeichnet, dass** der Behälter (3) wiederverschließbar ist.

10. Verpackungsset (1) nach einem der vorstehenden Ansprüche, wobei das Verpackungsset aus einem Schauständer (2) und einem Behälter (3) besteht.

11. Verpackungsset (1) nach einem der vorstehenden Ansprüche, wobei die Körperpflegeartikel Einwegabsorptionsartikel für die Damenhygiene, vorzugsweise Tampons, Slipeinlage, Damenbinde oder Inkontinenzeinlagen sind.

## Revendications

1. Trousse de conditionnement (1) pour des articles de soin personnel, **caractérisée en ce que** ladite trousse de conditionnement comprend un support de présentation (2) et au moins un récipient souple (3), ledit récipient (3) étant constitué d'un matériau essentiellement souple choisi parmi des matériaux en film ou non-tissés ou leurs combinaisons, ledit récipient pouvant être inséré de façon amovible dans ledit support de présentation (2), ledit récipient (3) ayant une dimension en longueur, une dimension en largeur et une dimension en épaisseur, ledit récipient (3) ayant en outre un intérieur creux comprenant plusieurs articles de soin personnel, ledit récipient (3) ayant au moins une position dressée dans laquelle la plus petite desdites dimensions dudit récipient (3) est orientée parallèle par rapport à une surface horizontale lisse, dans laquelle ledit récipient (3) n'est pas capable de maintenir ladite position dressée d'une manière autoporteuse, ledit support de présentation (2) étant adapté pour recevoir ledit récipient (3), ledit support de présentation (2) soutenant ledit récipient (3) pour maintenir ladite position dressée lorsque ledit récipient est inséré dans ledit support de présentation (2) dans laquelle ledit support de présentation (2) soutient ledit récipient (3) pour maintenir une position dans laquelle la plus grande desdites dimensions dudit récipient (3) est orientée verticalement par rapport à une surface horizontale lisse.

2. Trousse de conditionnement selon l'une quelconque des revendications précédentes, **caractérisée en ce que** ledit support de présentation comprend au moins une paroi latérale.

3. Trousse de conditionnement selon la revendication 3, **caractérisée en ce que** ladite paroi latérale a une hauteur, qui est plus basse que la plus grande dimension dudit récipient.

4. Trousse de conditionnement (1) selon l'une quelconque des revendications précédentes, **caractérisée en ce que** ledit support de présentation (2) est constitué d'un matériau relativement rigide, qui est de préférence choisi parmi le papier cartonné, le carton, carton blanchi, le papier renforcé, le carton ondulé ou similaires, ou un matériau polymère de type plastique ou caoutchouc, ou leurs combinaisons.

5. Trousse de conditionnement (1) selon l'une quelconque des revendications précédentes, **caractérisée en ce que** ledit support de présentation (2) comprend des zones à travers lesquelles le récipient (3) est visible.

6. Trousse de conditionnement (1) selon l'une quelconque des revendications précédentes, **caractérisée en ce que** ledit support de présentation (2) est pourvu d'un moyen améliorant l'aptitude à la mise au rebut en rendant ledit support de présentation (2) pliant, ledit moyen étant de préférence choisi parmi des lignes de faiblesse, des traits de coupe ou des joints adhésifs semi-permanents.

7. Trousse de conditionnement (1) selon l'une quelconque des revendications précédentes, **caractérisée en ce que** ledit récipient (3) est exempt de n'importe quelles informations permettant l'identification des produits qu'il contient et les informations relatives au produit doivent uniquement se trouver sur ledit support de présentation (2).

8. Trousse de conditionnement (1) selon l'une quelconque des revendications précédentes, **caractérisée en ce que** ledit récipient (3) comprend un moyen permettant de faciliter l'ouverture dudit récipient.

9. Trousse de conditionnement (1) selon l'une quelconque des revendications précédentes, **caractérisée en ce que** ledit récipient (3) est refermable.

10. Trousse de conditionnement (1) selon l'une quelconque des revendications précédentes, où ladite trousse de conditionnement est constituée d'un support de présentation (2) et d'un récipient (3).

11. Trousse de conditionnement (1) selon l'une quelconque des revendications précédentes, dans laquelle lesdits articles de soin personnel sont des articles absorbants jetables pour l'hygiène féminine, de préférence des tampons, un protège-slip, une serviette hygiénique ou des serviettes de protection contre les fuites urinaires.
